# EUROPEAN PATENT SPECIFICATION

(11) **EP 2 909 340 B1**
(45) Date of publication and mention of the grant of the patent: **02.08.2017**
(21) Application number: 13789899.5
(22) Date of filing: 18.10.2013
(51) Int. Cl.: C12Q 1/68

(54) **DIAGNOSTIC METHOD FOR PREDICTING RESPONSE TO TNF ALPHA INHIBITOR**
DIAGNOSEVERFAHREN ZUR VORHERSAGE DER REAKTION AUF TNF-ALPHA-INHIBITOR
PROCÉDÉ DE DIAGNOSTIC POUR PRÉDIRE UNE RÉPONSE À UN INHIBITEUR DE TNF ALPHA

(30) Priority: 19.10.2012 HU P1200607
(43) Date of publication of application: 26.08.2015
(73) Proprietor: Egis Gyógyszergyár Zrt., 1106 Budapest (HU)
(72) Inventor: NAGY, László, 4028 Debrecen (HU); MESKÓ, Bertalan, 4600 Kisvárda (HU); STEINER, László, 4028 Debrecen (HU); ZAHUCZKY, Gábor, 4241 Bocskaikert (HU); HOLLÓ, Zsolt, 2145 Kerepes (HU)
(74) Representative: Stolmár & Partner Patentanwälte PartG mbB
(86) International application number: PCT/HU2013/000101
(87) International publication number: WO 2014/060785

(56) References cited:
- EP-A1- 1 857 559
- WO-A1-2012/061620
- WO-A2-2004/091657
- WO-A2-2008/132176
- ANTONIO JULIÀ ET AL: "An Eight-Gene Blood Expression Profile Predicts the Response to Infliximab in Rheumatoid Arthritis", PLOS ONE, vol. 4, no. 10, 1 January 2009 (2009-01-01), page e7556, XP055015104, ISSN: 1932-6203, DOI: 10.1371/journal.pone.0007556
- LEQUERRÉ THIERRY ET AL: "Gene profiling in white blood cells predicts infliximab responsiveness in rheumatoid arthritis", ARTHRITIS RESEARCH AND THERAPY, BIOMED CENTRAL, LONDON, GB, vol. 8, no. 4, 3 July 2006 (2006-07-03), page R105, XP021020585, ISSN: 1478-6354, DOI: 10.1186/AR1924

## Description

### Field of the invention

The invention lies in the field of diagnostic methods. Disclosed are novel *in vitro* methods for predicting whether a patient would be responsive to a treatment with a TNFα inhibitor.

### Background of the invention

Tumor necrosis factor (TNF) promotes the inflammatory response, which in turn causes many of the clinical problems associated with autoimmune disorders such as rheumatoid arthritis, ankylosing spondylitis, Crohn's disease, psoriasis, hidradenitis suppurativa and refractory asthma. These disorders are sometimes treated by using a TNF inhibitor.

Rheumatoid arthritis (RA) is considered to be a chronic, systemic inflammatory autoimmune disorder that may affect many tissues and organs, but principally attacks flexible joints. RA is a painful and often disabling condition that can lead to the loss of mobility. While Crohn's disease is a type of inflammatory bowel disease (IBD) that may affect any part of the gastrointestinal tract from mouth to anus, causing a wide variety of symptoms. It primarily causes abdominal pain, diarrhea, vomiting or weight loss but may also cause complications outside the gastrointestinal tract.

The presently available treatments for the above diseases are based on patient populations as a whole. As a result, known treatments may lead to some patients cycling through ineffective treatments before identifying an effective therapy. Thus a need exists for personalized medicine to better treat TNF alpha related diseases (e.g. RA or IBD) and to identify effective treatment options for a given patient.

EP 1857 559 discloses an in vitro method for predicting whether a patient would be responsive to a treatment with a TNFα blocking agent, which method comprises determining the expression level of eight genes in a biological sample of the patient, wherein said genes are EPS15, HLA-DPB1, AKAP9, RASGRP3. MTCBP-1, PTNP12, MRPL22 andRPS28.

WO 2011/097301 discloses a method of predicting the responsiveness of a subject having rheumatoid arthritis (RA) to treatment with a TNFα inhibitor, the method comprising determining the presence of an HLA-DRB 1 shared epitope (HLA-DRB 1 SE) allele in a sample from the subject, wherein the presence of at least one copy of the HLA-DRB1 SE allele indicates that the subject will be responsive to treatment with the TNFα inhibitor.

Despite the fact that WO 2011/097301 and EP 1857 559 disclose methods for predicting responsiveness to treatment with a TNF-alpha inhibitor there remains a need for further more effective and precise methods to determine whether a patient having a TNF alpha related disease would respond to various treatment options.

### Brief description of the invention

Until the end of 2010, more than 2 000 000 patients worldwide have received treatment with anti-TNF-α biologic agents such as infliximab, adalimumab and etanercept in conditions such as rheumatoid arthritis (RA) or Crohn's disease (CD), among others but the efficacy of these are different (M.P. Karampetsou et al., QJM (2010) 103 (12): 917-928.).

The basic problem of monoclonal antibody (mAb) therapy in chronic inflammatory diseases can be summarized by the conclusions of two recent publications. 1) A significant percentage, approximately 30% of RA patients fail to respond to biologic therapy (Van Baarsen et al., Genes and Immunity 11,622-629). 2) Results from several large studies focusing on using biologic therapies in autoimmune diseases also indicate that efficacy may decline following cycling to a second TNF inhibitor (Rubbert-Roth et al., Arthritis Research & Therapy 2009).

Therefore, predicting whether the patient would respond to the therapy before the first or the second therapeutic option is an unmet need in the clinical setting and would have a large effect on the use of these medications by making treatment more cost-effective and providing patients the opportunity to receive personalized therapy.

The method of the invention is based on the use of bioinformatics based algorithm to identify sets of genes the combined expression profiles of which allow distinguishing between responder and non-responder patients to a treatment with a TNFα inhibitor.

More specifically, to solve the above problem present inventors developed an in vitro method for predicting whether a patient would be responsive to a treatment with a TNFα inhibitor, as defined in appended claims 1 to 7. Further disclosed herein is a method which method comprises determining the expression level of at least 6 genes selected from ABCC4, AIDA, ARHGEF12, BMP6, BTN3A2, CA2, CADM2, CD300E, CYP1B1, ENDOD1, FCGR1A, FMN1, GCLC, GPR34, HORMAD1, IGF2BP2, IL18R1, IL1RL1, KAT2B, MAP1LC3B, MMD, MS4A4A, MS4A7, ODC1, PBX1, PCYT1B, PIP4K2A, PIP5K1B, PRDM1, PSME4, RAD23A, RIOK3, RNASE2, RNF11, SLC7A5, THEM5, TMEM176A, TMEM176B, UBE2H, WARS genes or from APOBEC3A, AQP9, CCL4, CNTNAP3, CYP4F3, DHRS9, EIF2AK2, ELOVL7, EPSTI1, FCGR3A, GPAM, GPR15, GZMB, IFI35, IFI44, IFI44L, IFI6, IFIT1, IFIT2, IFIT3, IFITM1, IL2RB, IRF2, IRF7, MGAM, MICA, MME, MX1, OR2A9P, PF4, PTGS2, RAVER2, RFC1, RGS1, RSAD2, S100P, SERPINB10, SERPING1, SIGLEC1, TNF, TNFAIP6 in a biological sample of said patient. According to a preferred embodiment the relative expression level of the selected genes are determined compared to a housekeeping gene. Preferably said housekeeping gene is cyclophilin more preferably Cyclophilin A (PPIA). In the methods of the invention, the biological sample is peripheral blood more preferably the expression level is determined in peripheral blood mononuclear cells (PBMC).

According to a first preferred aspect of the disclosure the expression level of ELOVL7, IFI44L, IFIT1, IFIT3, MICA, OR2A9P and RAVER2 genes; or the expression level of APOBEC3A, IFI44, IFI44L, IFIT1, IFITM1, MICA and RGS1 genes; or the expression level of APOBEC3A, DHRS9, IFI35, IFI44, IFI44L, MICA and RFC1 genes are determined in said biological sample, preferably in a patient who has rheumatoid arthritis.

According to a second preferred aspect of the disclosure the expression level of BMP6, CD300E, CYP1B1, ODC1, RNF11 and UBE2H genes; or the expression level of ARHGEF12, CADM2, CD300E, GCLC, RIOK3 and UBE2H genes; or the expression level of CADM2, CD300E, CYP1B1, MMD, ODC1, RNF11 and UBE2H genes are determined in said biological sample, preferably in a patient who has Inflammatory Bowel Disease e.g. Crohn's disease.

According to a third preferred aspect the method is performed to follow the efficacy of said treatment with a TNFα inhibitor.

According to a preferred embodiment of the invention said TNFα inhibitor is an anti-TNFα antibody, a TNF fusion protein or a recombinant TNF binding protein, more preferably said TNFα inhibitor is Adalimumab, Certolizumab pegol, Etanercept, Golimumab, Infliximab, Pegsunercept or any biosimilar versions thereof , even more preferably said TNFα inhibitor is Infliximab or any biosimilar version thereof.

In another aspect the method of the invention further comprises the step of comparing the expression level of the above genes with reference values obtained from responder and non-responder groups of patients.

Preferably in the method of the invention the expression level is determined by quantifying the level of mRNA of said genes in the biological sample. DNA chip technology and reverse transcriptase-quantitative real time polymerase chain reaction (RT-QPCR) are particularly useful for determining the expression level of said genes.

According to a further preferred embodiment of the invention the method further comprises the step of determining the level of a biomarker protein. Preferably said biomarker protein is a pro-inflammatory cytokine, chemokine or an anti-drug antibody.

Furthermore the invention relates to TNFα inhibitor for use in the treatment of a TNFα related disease, wherein the treated patient was classified as responder to a treatment with a TNFα inhibitor by the method of the invention, preferably said TNFα inhibitor is Adalimumab, Certolizumab pegol, Etanercept, Golimumab, Infliximab or Pegsunercept.

### Brief description of the drawings

Figure 1: Timeline and design of the study of the invention.

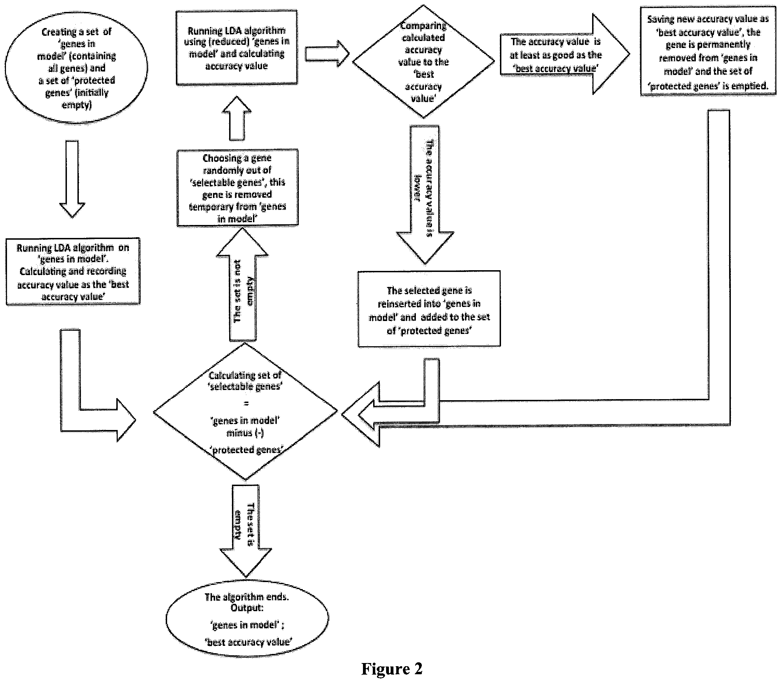

Figure 2: Schematic pathway of automatic gene panel generation.
Figure 3: Normalized mRNA levels of significantly changing genes in Rheumatoid arthritis (RA) patient groups before and after therapy (p values for AQP9: 0,02, TNFAIP6: 0,028, IGJ: 0,012). Data were calculated based on microarray measurements.
Figure 4: Normalized mRNA levels of the 4 genes found to be statistically significantly changing regarding NR (Non-responder) vs. R (Responder) comparison in RA.
Figure 5: Normalized mRNA levels of significantly changing genes in Crohn's disease (CD) patient groups before and after therapy (p values for MMP8: 0.018, AQP9: 0.011, IGKC: 0.001, TNFAIP6: 0.005, MGAM: 0.011) Data were calculated based on microarray measurements.
Figure 6: Normalized mRNA levels of the 4 genes found to be statistically significantly changing regarding NR vs. R comparison in CD.

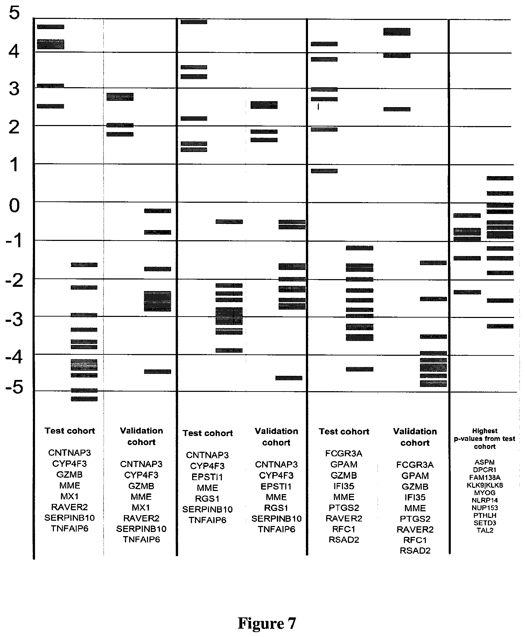

Figure 7: Three gene lists scored by linear discriminant analysis (LDA) in rheumatoid arthritis (RA). Bars on the left represent non-responders, bars on the right represent responders. The larger the distance between the groups and the smaller the overlap between samples, the higher the power of separation of the gene list is. The gene panel from the microarray experiment (test cohort) is on the left, the gene panel from the RT-QPCR experiments (validation cohort) is on the right for each gene panel, and a list of genes with the highest p values serving as a negative control based on the microarray data is on the right.

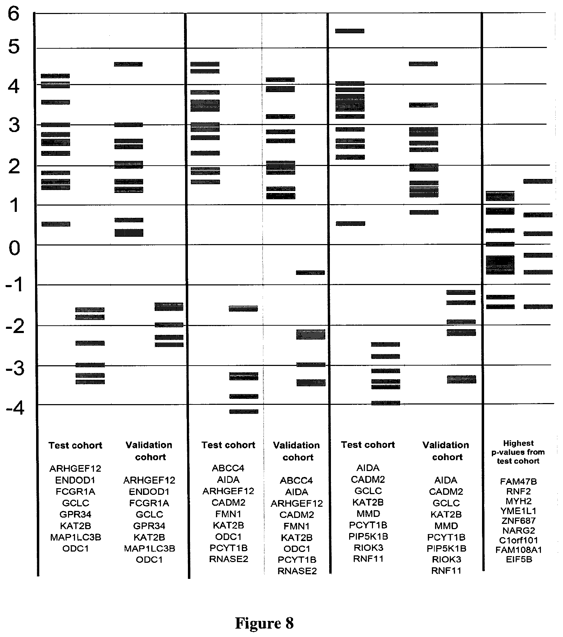

Figure 8: Three gene lists scored by linear discriminant analysis (LDA) in Crohn's disease (CD). Bars on the left represent non-responders, bars on the right represent responders. The larger the distance between the groups and the smaller the overlap between samples, the higher the power of separation of the gene list is. The gene panel from the microarray experiment (test cohort) is on the left, the gene panel from the RT-QPCR experiments (validation cohort) is on the right for each gene panel, and a list of genes with the highest p values serving as a negative control based on the microarray data is on the right.
Figure 9: Correlation between the number of genes in each gene panel and the minimum F value calculated for that panel either in the test or validation cohort in RA.
Figure 10: Correlation between the number of genes in each gene panel and the minimum F value calculated for that panel either in the test or validation cohort in CD.
Figure 11: IFNγ levels measured by ELISA in CD (test cohort)
Figure 12: IL-6 levels measured by ELISA in CD (test cohort)
Figure 13: Scatter plots of serum cytokines showing significant differences in RA (test cohort)
Figure 14: TNFα levels measured by ELISA in baseline RA samples from test cohort
Figure 15: TNFα levels measured by ELISA in week 2 RA samples from test cohort
Figure 16: TNFα levels measured by ELISA in baseline and week 2 RA samples from test cohort
Figure 17: Infliximab levels measured by ELISA in RA patients at week 2 and 14 (test cohort).
Figure 18: Infliximab levels at week 2 in CD patients measured by ELISA (test cohort)
Figure 19: Infliximab levels at week 2 in RA patients measured by ELISA (test cohort).

### Detailed description of the invention

Global gene expression profiling in peripheral blood is a proven technology in describing the pathogenetic background of autoimmune disorders and stratifying the diseases. Peripheral blood is an accessible source of biological material including cells and has clear advantages to use it for screening processes. Furthermore, as circulating peripheral blood mononuclear cells (PBMCs) are key cells of inflammation; several research groups examined PBMCs in microarray experiments. Gene expression patterns in circulating monocytes, T cells and B cells may reflect mechanisms of the disease, but not necessarily, which means that it is a challenge to extend pharmacogenomic markers to multigene diagnostic tests based on gene panels predicting response to therapies or disease progression. PBMC gene expression profiling provides a less expensive and less invasive alternative to biopsy or other invasive methods. However, up until now, the comparison of gene expression patterns of different autoimmune diseases focusing on a specific therapy has not been done.

Biomarkers or sets of combined biomarkers predicting response to therapy are now commonly used to improve the specificity of treatment. Using the least invasive peripheral blood sampling has also clear advantages. Although limitations include the sampling difficulty regarding the laboratory processing of samples, and in order to minimize heterogeneity of samples, strict guidelines have to be followed by both clinicians collecting and researchers processing the samples.

Present inventors determined gene panels with the most discriminatory power through global peripheral blood gene expression profiling in a test patient cohort and validated results on an independent patient cohort.

In summary the method of the invention in one aspect can be summarized as follows. Peripheral blood is taken from RA patients and optionally PBMC's are separated. From the peripheral blood or the isolated PBMC's RNA is isolated then reverse transcribed to cDNA. For the determination of relative expression levels of the selected genes according to the invention simultaneously RT-QPCR method is used.

The approach followed by the present inventors was to perform a global gene expression analysis in a test cohort with Affymetrix microarray technology in order to identify a list of genes that could be validated in an independent cohort with a more sensitive real-time (RT) qPCR method. RT-QPCR technology is the most robust tool for gene-expression measurements (in terms of sensitivity, dynamic range, standardization, throughput and price) that also makes it ideal as a diagnostic tool.

However, we supposed that not only baseline data, but data obtained at week 2 could be used for the validation. Thus genes determining responder status in a statistically significant way at week 2 were also added to the validation gene set; as well as a few genes from the relevant literature.

Regarding the comparisons of baseline and week 2 samples by microarray in both conditions, the effects of the therapy itself have been represented by 5 genes in CD out of which AQP9 and TNFAIP6 were earlier identified as IBD markers on the gene expression levels; MGAM was found to be a genomewide association predictor of response to TNFα blocking therapy in pediatric IBD, while the expression of MMP8 has been demonstrated in the actively inflamed area in the ulcer base of colonic samples in IBD, and its presence in stroma is suggestive of IBD etiology. In RA each gene that were found to be changing significantly in the first two weeks of therapy are relevant to its pathogenesis: AQP9 and TNFAIP6 discriminated RA patients from healthy controls through PBMC gene expression profiling; and IGJ (immunoglobulin J) showed significantly higher mRNA expression in twins with RA compared with their healthy co-twins.

Validation by RT-QPCR in an independent cohort also resulted in a list of genes with significant differences between responders and non-responders. In CD, these genes include TMEM176B and TMEM176A that are considered targets of dendritic cell function by forming multimers and restraining dendritic cell maturation; UBE2H regarding which TNF-a is a known regulator of the UBE2H-dependent ubiquitin conjugating activity; and WARS, a Tryptophanyl-tRNA synthetase. In RA, CYP4F3 that is associated with ostheoarthirtis pathomechanisms; DHRS9, MGAM; and PF4 was detected as a predictor of non-response for infliximab in RA in a proteomic study were found to be significant.

Although single genes might show significant differences between responders and non-responders, underlying differences accounting for a larger power of prediction can only be detected by analyzing gene panels.

To identify gene panels with the highest discriminatory power Canonical variates analysis (CVA) or Linear discriminant analysis (LDA) was used, because if compared to univariate analysis that may disregard potential interactions among genes, it can reveal underlying differences by using genes simultaneously as a gene panel providing perfect segregation in the multidimensional space.

It is known that sets of outcome-related gene panels identified by similar gene expression studies had only a few genes in common which might be attributed to the different methods of sample preparation, mRNA extraction or analysis of the data and, as well as individual variations and heterogeneities associated with markers, even in a clinically homogenous cohort of patients. Being included in the list of genes with statistically significant differences between responders and non-responders does not necessarily indicate the importance of the gene in the pathogenetics of the disease or therapy, accordingly the entire list of response-related genes should be analyzed in order to detect the potential targets for treatment.

Making automated the selection of gene panels with a high discriminatory power between responders and non-responders in both cohorts and diseases by building a bioinformatics-based algorithm resulted in about 4700 gene panels in each condition with a 100% segregation regarding responder status. Among them 3-3 gene panels were identified with the highest discriminatory power considering F values of the panels, cross-validation data and margins between the segregated groups.

Present inventors surprisingly have found that
1) in chronic inflammatory diseases, such as in CD and RA peripheral blood gene expression profiles are suitable for determining predictive gene panels the expression levels of which if measured prior to infliximab therapy identify patients who are susceptible to the therapy;
2) surprisingly entirely different gene panels are required for the prediction of the responder status in CD and RA despite the fact that these conditions have similar pathogenetic background; and
3) several gene panels were identified that show perfect segregation in the test and validation cohort as well as strong segregation in the cross validation analyses.

Sample collection is the crucial point in the method of the invention. One of the main criteria of the method applied that it should stabilize RNA, thereby making possible the storage and transfer of samples. Such methods are commercially available but they produce more or less different cell populations then that which was used in the study (PBMC/Trizol). Sample collection also has to be able to provide as a minimum 120-140 samples that is required for appropriate statistics. Sample processing, QC and RT-qPCR measurements are well-established technologies.

Besides the described PBMC/Trizol sample collection method we have tested other sample collection methods. Among these the PAXGene (Quiagen Corp., USA) and the LeukoLock (Life Technologies Corp., USA) sampling method showed good correlation with the PBMC/Trizol method. However in clinical practice the most preferred sampling method would be the PAXGene sampling method, as this method requires less laboratory equipment and skills from the person who does the sample collection.

The gene set according to the disclosure that was identified and validated by present inventors fulfills an unmet need for a genomic method discriminating unambiguously between responders and non-responders for a TNFα inhibitor (e.g. infliximab) therapy either in Crohn's disease (CD) or in rheumatoid arthritis (RA). The diagnostic method according to the invention gives an opportunity to introduce personalized healthcare first in this field that benefits all patients. Furthermore it is not only beneficial for patients who could be prevented from receiving an inefficient therapy and then cycling to the appropriate one, but clinicians, regulatory and reimbursing authorities and providers also profit from the increased efficacy and safety of the therapy.

### EXAMPLES

### Study Design

20 Crohn's disease (CD) and 19 rheumatoid arthritis (RA) patients were included in the study (sampling before and 2 weeks after therapy) in the **test cohort** for microarray experiments.

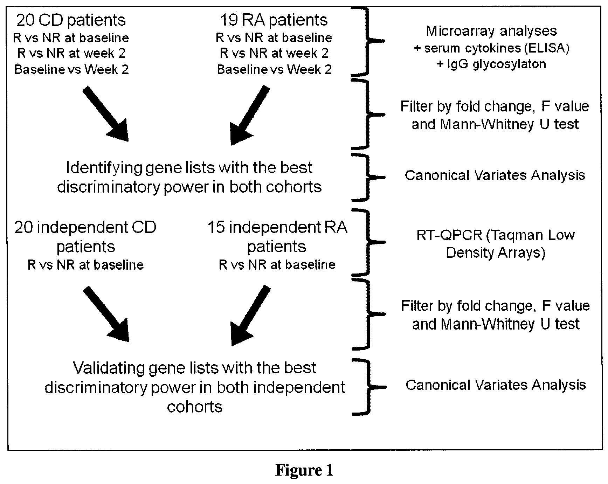

For the validation process, samples from 15 RA patients at week 0 **from the validation cohort,** 5 patients from the test cohort (for technical validation) and from 20 CD patients at week 0 **from the validation cohort** were included in the RT-QPCR experiments. The schematic diagram of figure 1 shows the timeline and design of the study.

### Patient recruitment

### A) Rheumatoid arthritis

### Inclusion criteria:

- Clinically diagnosed rheumatoid arthritis (criteria of the American Rheumatism Association from 1987)
- Age between 20 and 60
- A failure to respond to at least two disease modifying anti-rheumatic drugs including methotrexate
- Active disease (defined as having a disease activity score evaluated in 28 joints (DAS28) >3.2).
- Anti-TNF therapy naive patients
- Prednisone therapy 10mg per day was allowed provided that the dosage has been stable for at least 2 months before entry.
- Oral corticosteroids (maximum dose of 10mg per day - prednisone) and non-steroidal anti-inflammatory drug were allowed if stable for at least 1 month before baseline.
- Patients were on maximal tolerable methotrexate treatment (5-30mg per week), which had to be stable for at least 4 weeks before baseline.
- Women:Men ratio is 3:1

### Exclusion criteria:

smoker or ex-smoker; pregnancy or breastfeeding; current or recent malignome; clinically significant co-morbidities; active infectious disease; Patients with a history of an acute inflammatory joint disease of different origin

### Collected parameters:

Age, Diagnosis of RA (year), DAS28, CRP, We, RF, Co-morbidities, Drugs (e.g. DMARDS) After 14 weeks of treatment, the clinical response to treatment is assessed using both the EULAR criteria and the reduction in DAS28 of at least 1.2.

### B) Crohn's disease

### Inclusion criteria:

- Clinically diagnosed Crohn's disease
- Age between 20 and 60
- CDAI >250
- Never on anti-TNF therapy
- metothrexate (MTX) therapy, but <20 mg/week
- prednisolone therapy, but <10 mg/day
- Women:Men ratio is 1:1

### Exclusion criteria:

smoker or ex-smoker; pregnancy or breastfeeding; current or recent malignome; clinically significant co-morbidities; active infectious disease; Patients with a history of an acute inflammatory bowel disease of different origin

### Collected parameters:

Age, Diagnosis of CD (year), CDAI (if CDAI dropped below 150, patients were considered responders, otherwise non-responders), CRP, We, Co-morbidities, Drugs, Synopse of colon biopsy (if available), Responsiveness at week 14

### Patient Sample Collection, Processing and Storage (PBMC/Trizol)

Responsiveness to the therapy was determined 14 weeks after the therapy by clinicians based on the criteria described above. Peripheral blood samples were collected (10 ml) in Venous Blood Vacuum Collection Tubes containing EDTA (BD Vacutainer K2E) for PBMC separation and 10 ml peripheral blood in native tubes for the extraction of serum samples. All samples were processed within one hour after sample collection.

PBMCs were separated by Ficoll gradient centrifugation. Briefly, peripheral blood was diluted with 10 ml of physiological saline and layered on 10 ml of Ficoll. Centrifugation was performed on 2500 rpm for 20 minutes, and then layer of PBMCs was collected. Cells were washed with saline by twice (1700 rpm, 7 minutes) and lysed in Trizol reagent and stored at -70 C until RNA isolation.

### Statistics of clinical parameters

Clinical parameters of patient cohorts were compared by using GraphPad Prism, and Mann-Whitney U test was used (p<0,05 was considered statistically significant).

### RNA isolation

RNA was isolated from peripheral blood mononuclear cells (PBMC) using Trizol reagent (Invitrogen) according to manufacturer's protocol. RNA quantity and quality were checked on UV photometer NanoDrop 1000 (Thermo Scientific) instrument and Agilent BioAnalyzer (Agilent Technologies).

### Microarray

Affymetrix GeneChip Human Gene 1.0 ST array was used to analyse global expression pattern of 28869 well-annotated genes. Ambion WT Expression Kit (Applied Biosystems) and GeneChip WT Terminal Labeling and Control Kit (Affymetrix) were used for amplifying and labeling 250 ng of RNA samples. Samples were hybridized at 45 degrees Celsius for 16 hours and then standard washing protocol was performed using GeneChip Fluidics Station 450 and the arrays were scanned on GeneChip Scanner 7G (Affymetrix).

### Microarray data analysis

Microarray data were analyzed with Genespring GX10 (Agilent Biotechnologies). Affymetrix data files were imported using RMA algorithm and median normalization was performed. Regarding the baseline vs week 2 samples comparison, 20% of probe sets with the lowest expression levels were filtered out in the first step, then the list of remaining probe sets was filtered by fold change (1.2 fold cut off) and statistical analysis was performed using paired Mann-Whitney U-test with Benjamini-Hochberg multiple-testing correction.

Regarding the responder vs. non-responder comparison, 20% of probe sets with the lowest expression levels were filtered out in the first step, then the list of remaining probe sets was filtered by fold change (1.2 fold cut off) and statistical analysis was performed using unpaired T-test with Benjamini-Hochberg correction for multiple-testing. Functional categorization of genes was performed with Panther Classification System (http://www.pantherdb.org/).

### RT-QPCR

Gene expression data was obtained using TaqMan Low Density Array (TLDA) (Applied Biosystems) which is a 384-well micro fluidic card that enables to perform 384 simultaneous real-time PCR runs and which has been used for gene expression profiling in several studies. This low- to medium- throughput micro fluidic card allows for 2 samples to be run in parallel against 96 TaqMan® Gene Expression Assay targets that are preloaded into each of the wells on the card. cDNA was generated with High Capacity cDNA Reverse Transcription Kit according to manufacturer's protocol. 1 micrograms of RNA were used per sample in the RT-PCR runs. 400 ng (4 µl) cDNA was used in each sample. 196 µl nuclease free water and 200 µl 2× TaqMan Universal PCR Master Mix (Applied Biosystems) were added for the Real-Time Quantitative PCR measurements. This mixture was then equally divided over four sample-loading ports of the TLDA, each connected to one set of the 96 genes of interest. The arrays were centrifuged once (1', 1300 RPM on room temperature) to equally distribute the sample over the wells. Subsequently, the card was sealed to prevent an exchange between wells. RT-QPCR amplification was performed using an Applied Biosystems Prism 7900HT sequence detection system with the following thermal cycler conditions: 2 min at 50°C and 10 min at 94.5°C, followed by 40 cycles of 30 s at 97°C and 1 min at 59.7°C. 91 genes were chosen based on our previous microarray experiment and the remaining 5 genes were housekeeping genes for normalization.

### RT-QPCR data analysis

RT-QPCR data files were imported Data Assist software (Applied BioSystems) and raw data were normalized by ΔCt method. Cyclophilin A (PPIA) was chosen as normalizer gene because its expression showed the less variation between samples.. To find differentially expressed genes between the responder and non-responder groups non-parametric statistical test (Mann-Whitney U test) and Canonical Variate Analysis (CVA) were done.

### Canonical variate analysis (CVA) or Linear discriminant analysis (LDA)

Separation between predefined groups of objects is best revealed by Canonical variate analysis (CVA). CVA is the generalization of Linear discriminant analysis (LDA), the two terms are used equivalently in the study. CVA was used to determine whether the groups of responders and non-responders are separable in the multidimensional space spanned by the genetic variables, and if so, which gene subsets have the best discriminatory power. The results of CVA are the so-called canonical scores obtained from the canonical functions derived through eigenanalysis, which serve as coordinates of observations in the canonical space.

### Automatic gene panel generation

Linear discriminant analysis (LDA) (Hamadeh HK et al. Prediction of compound signature using high density gene expression profiling. Toxicol Sci. 2002 Jun;67(2):232-40.) was used for automatically generating gene panels that show 100% segregation between responders and non-responders in both conditions and in both cohorts (test and validation) according to the following algorithm (40 genes in CD and 41 genes in RA were used that were pre-filtered during the experiments with the test cohorts and validated in the validation cohorts.):
1) The set of 'genes in model' is created. Initially, this set contains all genes. A set of genes with so-called 'protected genes' is also created. Initially, this set is empty.
2) F-value that is the ratio of between-group variability and within-group variability is calculated for each gene.
3) The classifier algorithm (LDA) is run using the set of 'genes in model' both in test and validation cohorts. In both cases an accuracy percentage value is recorded as the 'best accuracy values'.
4) The set of 'selectable genes' is defined as:
   'selectable genes':= 'genes in model' minus 'protected genes'
   If the group of 'selectable genes' is not empty then the algorithm is continued in step 5, else the algorithm skips to step 7.
5) Genes are selected from the set of 'selectable genes' according to the following models:
   a) randomly with equal probabilities (**uniform model**);
   b) randomly with a probability that is inversely proportional to their F-value **(F_prop model**);
   c) genes with the lowest F-values (**min F model**).
   In either case, the selected gene is temporary removed from the set of 'genes in model'.
   The advantage of using stochastic models instead of min F model is that those can provide better segregation of patient groups. Uniform and F_prop models represent stochastic algorithms while min F model is deterministic.
6) The classifier is run using the (temporary reduced) set of 'genes in model'.
   a) If any of the two accuracy percentage values becomes lower, the selected gene is reinserted into the set of 'genes in model' and added to the set of 'protected genes'.
   b) If both accuracy percentage values are at least as good as the 'best accuracy values', the selected gene is permanently removed from 'genes in model' and the set of 'protected genes' is emptied. The 'best accuracy values' are overwritten with the calculated accuracy values.
   The algorithm returns to step 4.
7) The algorithm ends. The outputs are the set of 'genes in model' and 'best accuracy values'. Linear discriminant analysis was performed by using R software (R Development Core Team (2008). R: A language and environment for statistical computing. R Foundation for Statistical Computing, Vienna, Austria. ISBN 3-900051-07-0, URL http://www.R-project.org.) with package MASS (Venables, W. N. & Ripley, B. D. (2002) Modem Applied Statistics with S. Fourth Edition. Springer, New York) and package wordcloud (Ian Fellows (2012). wordcloud: Word Clouds. R package version 2.0. http://CRAN.R-project.org/package=wordcloud) for visualization.

### Enzyme-linked immunosorbent assays (ELISA)

To determine serum levels of IL-6, IL-8, IL-12, IFNg, TNFa, infliximab and anti-infliximab antibody, enzyme-linked immunosorbent assays (ELISA) were performed. Quantikin ELISA kits (R&D Systems) were used for IL-6, IL-8, IL-12 and IFNg measurements according to manufacturer's protocol. The levels of TNFa, infliximab and anti-infliximab antibodies were measured by LISA -TRACKER Premium Infliximab kit (BioMedical Diagnostics). The results were given in pg/ml. Data were analyzed by using GraphPad Prism, and Mann-Whitney U test was used (p<0,05 was considered statistically significant).

### EXAMPLES

### Example 1

### Global gene expression analysis of rheumatoid arthritis test cohort samples

### Rheumatoid arthritis test cohort patient groups

Samples from 19 patients at baseline and week 2 were included in the microarray experiments. 6 responders and 13 non or moderate responders were identified by clinicians. Each patient had the same basic therapy and was non-smoker. Between the non-responder (NR) and responder (R) groups, there were no significant differences regarding age, DAS28, HAQ, CRP, Rheumatoid factor, anti-CCP or DMARDs (Table 1).

**Table 1: Cumulated clinical parameters of patient groups in RA in the test cohort. NS means non-significant, DAS28, HAQ and DMARDs are score data.**

| | | Responders (ACR70-50) | Non- or moderate Responders (ACR20-0) |
|---|---|---|---|
| At week 0 | | 6 | 13 |
| Gender | Male/Female | 1/5 | 2/11 |
| Age | NS | 44,33 | 47,08 |
| DAS28 | NS | 5,63 | 5,26 |
| HAQ | NS | 1,27 | 2,06 |
| CRP (mg/l) | NS | 16,83 | 28,31 |
| DMARDs | NS | 2,83 | 2,69 |
| RF (IU/ml) | NS | 105,83 | 148,62 |
| CCP (IU/ml) | NS | 675,78 | 756,31 |

### Microarray analyses

Global gene expression profiling revealed genes with statistically significant differences between responders and non-responders at baseline. Analyzing samples obtained at week 2 showed genes with statistically significant differences between responders and non-responders. The expression of some genes was significantly different even at baseline (EPSTI1, IFI44, IFIT1, IFIT2, IFIT3, RFC1 and RSAD2); while others expressed differently at week 2 (FCGR3A, GPAM, MICA, ELOVL7, PF4, RGS1 and SNORD41). Many of these genes have relation to RA according to the literature (FCGR3A, MICA, PF4, IFIT1, etc.)

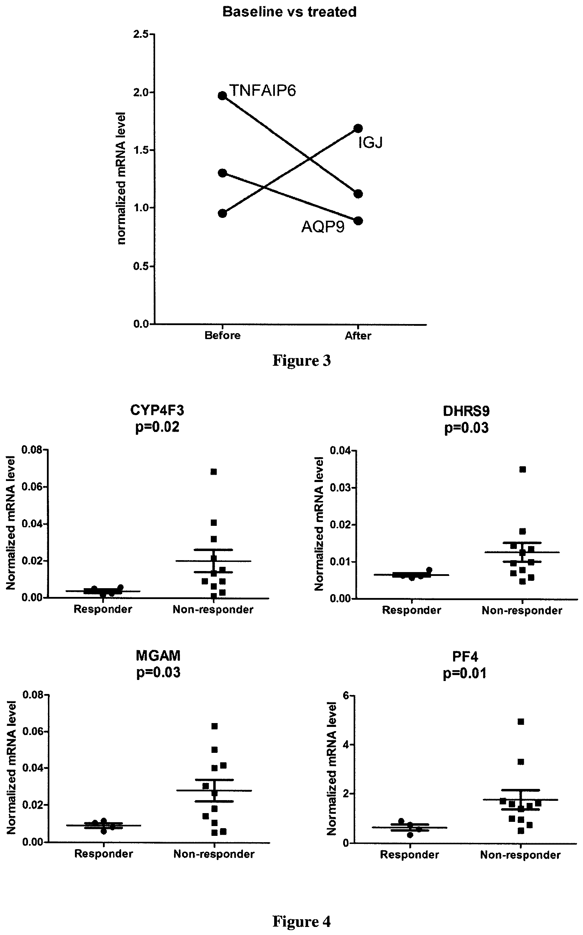

Comparing baseline and week 2 samples resulted in **a list of 3 genes (AQP9, IGJ and TNFAIP6) with statistically significant differences** even with Benjamini Hochberg correction for multiple testing. These genes represent the effects of the therapy over time (Figure 3).

### Example 2

### RT-QPCR validation of rheumatoid arthritis gene panels

### Rheumatoid arthritis validation cohort patient groups

4 responders and 11 non or moderate responders were identified by clinicians. Each patient had the same basic therapy and was non-smoker. Between the non-responder (NR) and responder (R) groups, there were no significant differences regarding age, DAS28, HAQ, CRP or DMARDs (Table 2).

**Table 2: Cumulated clinical parameters of patient groups in RA in the validation cohort. In the brackets the data of test cohort are also shown. DAS28, HAQ and DMARDs are score data.**

| | | Responders | Non- or moderate responders |
|---|---|---|---|
| At week 0 | (test cohort) | 4 (6) | 11 (13) |
| Gender | Male/Female | 0/4 (1/5) | 3/8 (2/11) |
| Age | Non-significant | 54 (44,33) | 56,2 (47,08) |
| DAS28 | Non-significant | 5,23 (5,63) | 5,44 (5,26) |
| HAQ | Non-significant | 1,59 (1,27) | 1,93 (2,06) |
| CRP (mg/l) | Non-significant | 18,9 (16,83) | 9,58 (28,31) |
| DMARDs | Non-significant | 3 (2,83) | 2,7 (2,69) |

### RT-QPCR analyses

Samples from 15 patients at week 0 and 5 patients from the test cohort for technical validation - 2 responders, 3 non-responders were included in the RT-QPCR experiments. The configuration of validation q-PCR assays were made as follows: based on the test cohort microarrays 29 probe sets proved to be statistically significant at baseline regarding the non-responder vs. responder comparison, out of which 27 genes (genes without annotation and small nucleolar RNAs were excluded) were included in the TLDA cards as well as 6 genes from the NR vs R comparison at week 2, and 10 genes were selected from the literature. Out of this, 41 genes were used in the final analysis as described below (genes showing no differences were excluded). RT-QPCR experiments resulted in **4 genes showing statistically significant** (1 tailed Mann-Whitney U test) differences between NRs and Rs (Figure 4). Technical validation was also preformed and proved to be successful.

### Example 3

### Global gene expression analysis of Crohn's disease test cohort samples

### Crohn's disease test cohort patient groups

Samples from 20 patients at baseline and week 2 were included in the microarray experiments (test cohort). 14 responders and 6 non-responders were identified by clinicians. Each patient had the same basic therapy and was non-smoker. Between the non-responder (NR) and responder (R) groups, there were no significant differences regarding age, CDAI, CRP, hemoglobin, leukocytes or neutrophils (Table 3).

**Table 3: Cumulated clinical parameters of patient groups in CD in the test cohort. CDAI, Crohn's Disease Activity Index is a score**

| | | Responders | Non-responders |
|---|---|---|---|
| At Week 0 | | 14 | 6 |
| Gender | Male/Female | 8/6 | 3/3 |
| Age | Non-significant | 36,2 | 36 |
| CDAI | Non-significant | 319,6 | 351,5 |
| CRP (mg/l) | Non-signifcant | 22,78 | 13,59 |
| Hemoglobin (g/l) | Non-significant | 125,1 | 120,6 |
| Leukocytes (G/l) | Non-significant | 9,02 | 8,01 |
| Neutrophils (%) | Non-significant | 70,0 | 74,5 |

### Microarray analyses

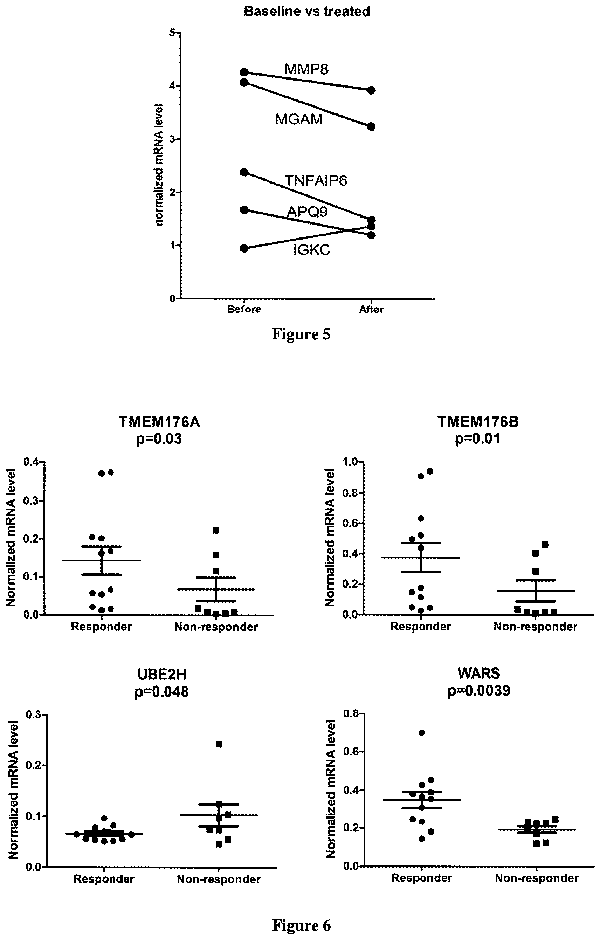

Global gene expression profiling revealed genes with statistically significant differences between responders and non-responders at baseline. Analyzing samples obtained at week 2 showed genes with statistically significant differences between responders and non-responders. Some of these genes were significantly changing one at baseline as well (DDX11L2, BMP6, THEM5 and ABCC4); while others were new findings at week 2 (GPR34, PRDM1, IL1RL1, CA2, MMD, SCL7A5, CADM2 and RAD23A). Many of these genes have relation to CD according to the literature (BMP6, ABCC4, CA2, IL1RL1 and PRDM1). Comparing baseline and week 2 samples resulted in a list of 5 genes (AQP9, IGKC, MGAM, MMP8 and TNFAIP6) with statistically significant differences. These genes represent the effects of the therapy over time (Figure 5).

### Example 4

### RT-QPCR validation of Crohn's disease gene panels

### Crohn's disease validation cohort patient groups

Samples from 20 patients at baseline were included in the RT-QPCR experiments (validation cohort). 13 responders and 7 non-responders were identified by clinicians. Each patient had the same basic therapy and was non-smoker. Between the non-responder (NR) and responder (R) groups, there were no significant differences regarding age, CDAI, CRP, hemoglobin, leukocytes or neutrophils (Table 4).

**Table 4: Cumulated clinical parameters of patient groups in CD in the validation cohort. In brackets the data of test cohort are also shown. CDAI, Crohn's Disease Activity Index is a score. RT-QPCR analyses**

| | | Responders | Non-responders |
|---|---|---|---|
| At baseline | (test cohort) | 13 (14) | 7 (6) |
| Gender | Male/Female | 8/5 (8/6) | 4/4 (4/3) |
| Age | Non-significant | 26,8 (36,2) | 30,2 (36) |
| CDAI | Non-significant | 338,3 (319,6) | 370.7 (351,5) |
| CRP (mg/l) | Non-significant | 27,2 (22,78) | 16,5 (13,59) |
| Hemoglobin (g/l) | Non-significant | 130 (125,1) | 127 (120,6) |
| Leukocytes (G/l) | Non-significant | 7,58 (9,02) | 9,24 (8,01) |
| Neutrophils (%) | Non-significant | 74,0 (70,0) | 72,83 (74,5) |

The configuration of validation q-PCR assays was made as follows: based on the 40 microarrays in the test cohort, 49 probe sets proved to be statistically significant at baseline regarding the non-responder vs. responder comparison, out of which 36 genes (genes without annotation and small nucleolar RNAs were excluded) were included in the TLDA cards as well as 8 genes from the NR vs. R comparison at week 2, and 7 genes from the literature. Out of this, 40 genes were used in the final analysis as described below (genes showing no differences were excluded). RT-QPCR experiments resulted in 4 genes showing statistically significant (1 tailed Mann-Whitney u test) differences between NRs and Rs (Figure 6).

### Example 5

### Biostatistical analysis of expression data

Statistical analysis was performed using gene expression data of the pre-filtered 40/41 (RA/CD) genes in both cohorts using automatic gene panel generation described above in details, an algorithm was designed for finding the best gene panels discriminating between responders and non-responders.

The algorithm was run with the deterministic **min_F** and 5000 times with both stochastic models. In RA **F_prop** produced 4747 combinations of gene panels showing 100% segregation between responders and non-responders both in the test and validation cohorts (using microarray and RT-QPCR data, respectively), while **uniform** produced even more, 4909 gene panels. In CD these numbers were 4657 for **F_prop** and 4878 for **uniform. min_F** model also produced 100% segregation but it has to be noted that stochastic models produced much more profound segregation in terms of accuracy indicators in both diseases. The high number of gene panels providing 100% segregation generated by 5000 runs suggests that there are other panels with 100% segregation, the total number of these panels is estimated exceeding 50 000.

Cross-validation is a way to predict the fit of a model to a hypothetical validation set when an explicit validation set is not available. We used leave-one-out cross-validation (LOOCV) that involves using a single observation from the original sample as the validation data, and the remaining observations as the training data. This is repeated such that each observation in the sample is used once as the validation data. For visualization 3-3 gene panels with the best discriminatory power were chosen considering F values, cross-validation data and margins between the segregated groups. A list of genes with the highest p values in the microarray experiment served as negative controls showing no segregation (Table 5).

The gene panel for RA with the best discriminatory power included genes such as CNTNAP3, CYP4F3, GZMB, MME, MX1, RAVER2, SERPINB10 and TNFAIP6 (RA1); while the second gene panel contained CNTNAP3, CYP4F3, EPSTI1, MME, RGS1, SERPINB10 and TNFAIP6 (RA2); the third one consisted of FCGR3A, GPAM, GZMB, IFI35, MME, PTGS2, RAVER2, RFC1and RSAD2 (RA3). (Figure 7).

The gene panel for CD with the best discriminatory power included genes such as ARHGEF12, ENDOD1, FCGR1A, GCLC, GPR34, KAT2B, MAP1LC3B and ODC1 (CD1); while the second gene panel contained ABCC4, AIDA, ARHGEF12, CADM2, FMN1, KAT2B, ODC1, PCYT1B and RNASE2 (CD2); the third one consisted of AIDA, CADM2, GCLC, KAT2B, MMD, PCYT1B, PIP5K1B, RIOK3 and RNF11 (CD3). (Figure 8)

**Table 5: Accuracy indicators of the gene panels selected for visualization . Segregation and cross validation data are presented of each selected gene panel and the negative control, as well.**

| **Gene panel #** | **RA1** | | **RA2** | | **RA3** | | **Negative control** |
|---|---|---|---|---|---|---|---|
| **Rheumatoid Arthritis cohort** | **Test cohort** | **Valid. cohort** | **Test cohort** | **Valid. cohort** | **Test cohort** | **Valid. cohort** | **Test cohort** |
| Segregation of the groups | 100% | 100% | 100% | 100% | 100% | 100% | 53% |
| Result of cross validation | 100% | 87% | 95% | 93% | 95% | 87% | - |

| Source of data | Microarray | RT-QPCR | Microarray | RT-QPCR | Microarray | RT-QPCR | Microarray |
|---|---|---|---|---|---|---|---|
| **Gene panel #** | **CD1** | | **CD2** | | **CD3** | | **Negative control** |
| **Crohn's disease cohort** | **Test cohort** | **Valid. cohort** | **Test cohort** | **Valid. cohort** | **Test cohort** | **Valid. cohort** | **Test cohort** |
| Segregation of the groups | 100% | 100% | 100% | 100% | 100% | 100% | 55% |
| Result of cross validation | 95% | 80% | 90% | 85% | 90% | 80% | - |
| Source of data | Microarray | RT-QPCR | Microarray | RT-QPCR | Microarray | RT-QPCR | Microarray |

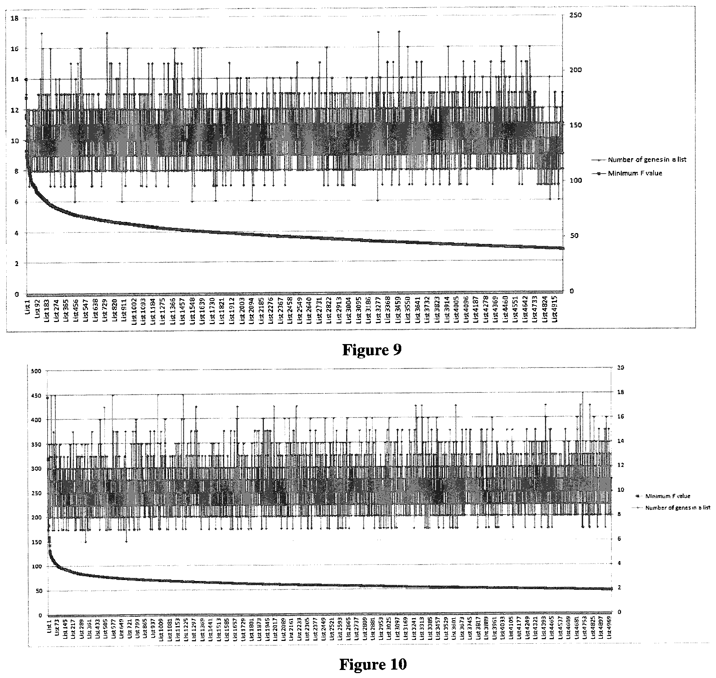

In figures 9 and 10 the number of genes within each panel showing a segregation of 100% between responders and non-responders and the smallest F value calculated for each patient group (the higher the F value is, the better the segregation is meaning that selecting the minimum F value represents the weakest point of the model) are shown sorted by F value in order to reveal the estimated number of gene panels with the highest discriminatory power. The inflection point of the minimum F value curve shows that in RA approximately 350 while in CD 200 gene panels resulted in a strong discrimination, noting that all gene panels on the plot led to a 100% differentiation between responders and non-responders.

### Example 6

### ELISA analysis

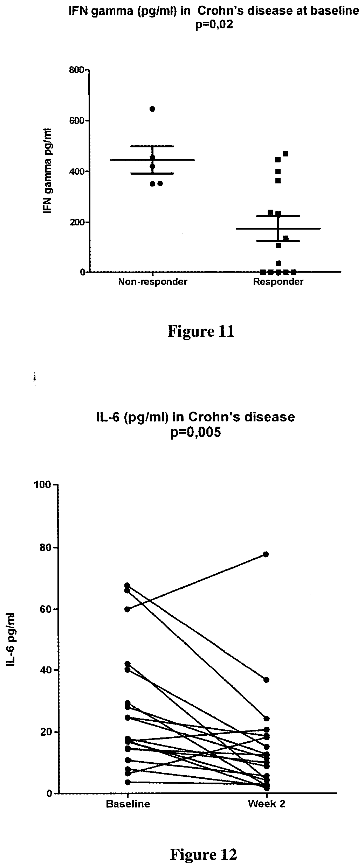

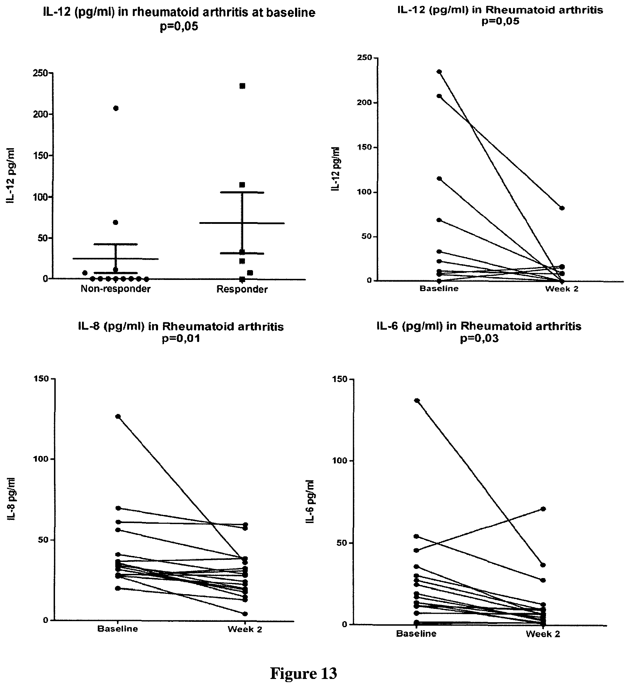

ELISA measurements were performed to determine serum protein levels of five pro-inflammatory cytokines (TNFa, IL6, IL8, IFNg and IL12), the therapeutic monoclonal antibody infliximab and the anti-drug (anti-infliximab) antibody. Table 6 shows that based on statistical analysis significant difference was found in RA between responders and non-responders at baseline in the level of IL-12, while in CD the level of IFNγ turned to be statistically different. TNFα was measured with TRACKER Premium Infliximab kit in RA cohort. Detailed scatter plots of the **significant** comparisons are shown in figures 11 - 13.

**Table 6: Statistics of cytokine ELISA measurements p-values were obtained by the statistical analysis of cytokine level data of NR vs. R and baseline vs. week 2 groups using Mann Whitney U test. Bold numbers represent statistically significant values.**

| | **Rheumatoid arthritis** | | **Crohn's disease** | |
|---|---|---|---|---|
| | **NR vs R at Baseline** | **Baseline vs Week 2** | **NRvsRat Baseline** | **Baseline vs Week 2** |
| **IL6** | 0,2 | **0**,**03** | 0,2 | 0,005 |
| **IL8** | 0,3 | **0**,**01** | 0,9 | 0,06 |
| **IL12** | **0,05** | **0,05** | 0,6 | 0,8 |
| **IFNg** | 0,5 | 0,5 | **0,02** | 0,06 |
| **TNF** | 0,2 | 0,67 | | |

TNFα, infliximab and anti-infliximab ELISA trial experiments were also performed to test LISA -TRACKER Premium Infliximab kit. As for the limited amount of the kit only samples from the test cohorts could be included. The following samples were included:

| | | | |
|---|---|---|---|
| **TNFα** | 17 RA at baseline, | 17 RA at week 2 | 7 RA at week 14 |
| **Infliximab** | 16 CD at week 2 | 19 RA at week 2 | 7 RA at week 14 |
| **Anti-infliximab** | 17 CD at week 2 | 19 RA at week 2 | 7 RA at week 14 |

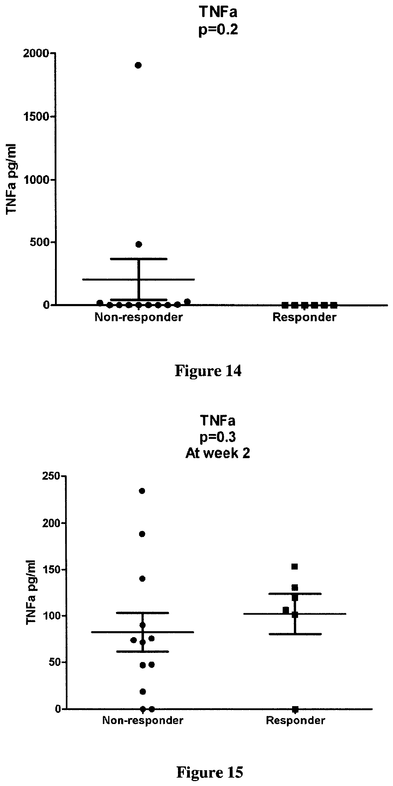

Therefore, TNFα was only measured in samples from RA patients of the test cohort but no difference could be detected between responders and no-responders either at baseline or at week 2 or at baseline versus week 2 comparisons (Figures 14 - 16).

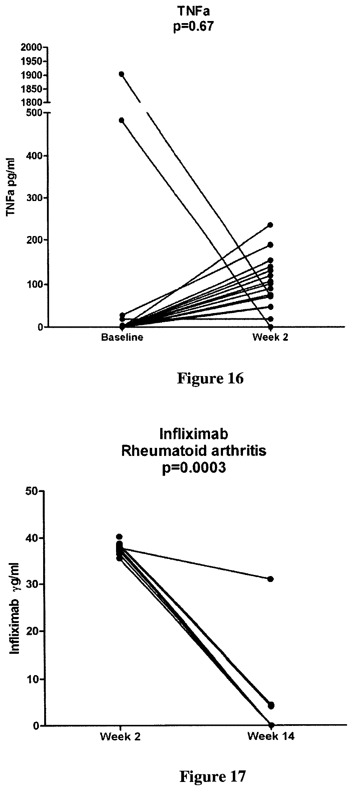

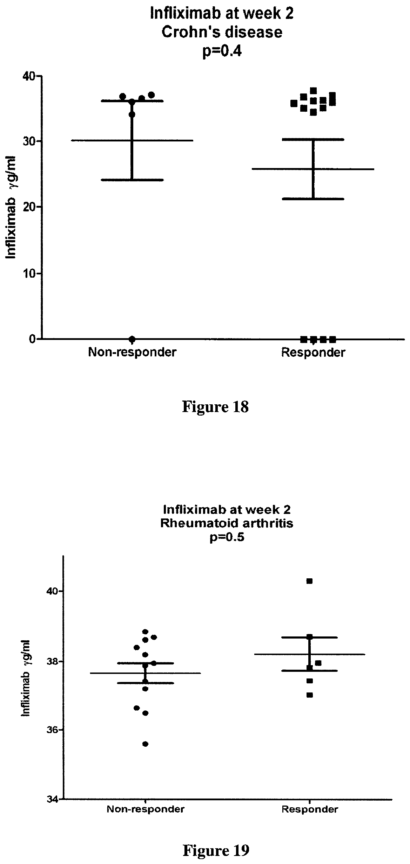

Infliximab and anti-infliximab were measured in week 2 and week 14 samples of RA and CD (test cohort) (Figures 17 - 19). Anti-infliximab levels could be only detected in 3 samples representing patients that showed zero infliximab levels at week 14.

## Claims

1. An in vitro method for predicting whether a patient having rheumatoid arthritis would be responsive to a treatment with a TNFα inhibitor, which method comprises determining the expression level of CNTNAP3, CYP4F3, GZMB, MME, MX1, RAVER2, SERPINB10 and TNFAIP6 genes, or the expression level of CNTNAP3, CYP4F3, EPSTI1, MME, RGS1, SERPINB10 and TNFAIP6 genes, or the expression level of FCGR3A, GPAM, GZMB, IFI35, MME, PTGS2, RAVER2, RFC1 and RSAD2 genes in a blood sample of said patient.

2. The method of claim 1, wherein the relative expression levels of the selected genes are determined compared to a housekeeping gene.

3. The method according to any of the preceding claims wherein the TNFα inhibitor is an anti-TNFα antibody, a TNF fusion protein or a recombinant TNF binding protein.

4. The method according to any of the preceding claims wherein the TNFα inhibitor is Adalimumab, Certolizumab pegol, Etanercept, Golimumab, Infliximab or Pegsunercept.

5. The method according to any of the preceding claims further comprising the step of comparing the expression level of said genes with reference values obtained from responder and non-responder groups of patients.

6. The method according to any of the preceding claims, wherein the expression level is determined by quantifying the level of mRNA of said genes in the blood sample.

7. The method according to any of the preceding claims, further comprising the step of determining the level of a biomarker protein.

## Patentansprüche

1. In vitro-Verfahren zur Vorhersage, ob ein Patient mit rheumatoider Arthritis auf eine Behandlung mit einem TNFα-Inhibitor reagieren würde, wobei das Verfahren die Bestimmung des Expressionsniveaus von CNTNAP3-, CYP4F3-, GZMB-, MME-, MX1-, RAVER2-, SERPINB10- und TNFAIP6-Genen, oder des Expressionsniveaus von CNTNAP3-, CYP4F3-, EPSTI1-, MME-, RGS1-, SERPINB10- und TNFAIP6-Genen oder des Expressionsniveaus von FCGR3A-, GPAM-, GZMB-, IFI35-, MME-, PTGS2-, RAVER2-, RFC1- und RSAD2-Genen in einer Blutprobe des Patienten umfasst.

2. Verfahren nach Anspruch 1, wobei die relativen Expressionsniveaus der ausgewählten Gene im Vergleich zu einem Housekeeping-Gen bestimmt werden.

3. Verfahren nach einem der vorhergehenden Ansprüche, wobei der TNFα-Inhibitor ein Anti-TNFα-Antikörper, ein TNF-Fusionsprotein oder ein rekombinantes TNF-bindendes Protein ist.

4. Verfahren nach einem der vorhergehenden Ansprüche, wobei der TNFα-Inhibitor Adalimumab, Certolizumab pegol, Etanercept, Golimumab, Infliximab oder Pegsunercept ist.

5. Verfahren nach einem der vorhergehenden Ansprüche, ferner umfassend den Schritt des Vergleichens des Expressionsniveaus dieser Gene mit Referenzwerten, erhalten von Responder- und Nicht-Responderpatientengruppen.

6. Verfahren nach einem der vorhergehenden Ansprüche, wobei das Expressionsniveau durch Quantifizierung des Niveaus der mRNA dieser Gene in der biologischen Probe bestimmt wird.

7. Verfahren nach einem der vorhergehenden Ansprüche, ferner umfassend den Schritt des Bestimmens des Niveaus eines Biomarkerproteins.

## Revendications

1. Procédé in vitro de prédiction du fait qu'un patient ayant une polyarthrite rhumatoïde réponde à un traitement avec un inhibiteur de TNFα, lequel procédé comprend déterminer le niveau d'expression des gènes CNTNAP3, CYP4F3, GZMB, MME, MX1, RAVER2, SERPINB10 et TNFAIP6, ou le niveau d'expression des gènes CNTNAP3, CYP4F3, EPSTI1, MME, RGS1, SERPINB10 et TNFAIP6, ou le niveau d'expression des gènes FCGR3A, GPAM, GZMB, IFI35, MME, PTGS2, RAVER2, RFC1 et RSAD2 dans un échantillon sanguin dudit patient.

2. Procédé selon la revendication 1, dans lequel les niveaux d'expression relatifs des gènes sélectionnés sont déterminés par rapport à un gène domestique.

3. Procédé selon l'une quelconque des revendications précédentes, dans lequel l'inhibiteur de TNFα est un anticorps anti-TNFα, une protéine de fusion au TNF ou une protéine de liaison au TNF recombinée.

4. Procédé selon l'une quelconque des revendications précédentes, dans lequel l'inhibiteur de TNFα est l'adalimumab, le certolizumab pegol, l'étanercept, le golimumab, l'infliximab ou le pegsunercept.

5. Procédé selon l'une quelconque des revendications précédentes, comprenant en outre l'étape de comparaison du niveau d'expression desdits gènes à des valeurs de référence obtenues à partir de groupes de patients répondeurs et non répondeurs.

6. Procédé selon l'une quelconque des revendications précédentes, dans lequel le niveau d'expression est déterminé en quantifiant le niveau d'ARNm desdits gènes dans l'échantillon sanguin.

7. Procédé selon l'une quelconque des revendications précédentes, comprenant en outre l'étape de détermination du niveau d'une protéine de biomarqueur.
